# EUROPÄISCHE PATENTANMELDUNG

(11) **EP 1 995 304 A1**
(43) Veröffentlichungstag der Anmeldung: **26.11.2008**
(21) Anmeldenummer: 08009561.5
(22) Anmeldetag: 26.05.2008
(51) Int. Cl.: C12M 1/00, C12M 1/107

(54) **Verfahren und Vorrichtungen zur wirtschaftlichen Herstellung von Bioölen**

(30) Priorität: 24.05.2007 DE 102007024187
(71) Anmelder: Pilema techn. Produkte GmbH, 06217 Merseburg (DE)
(72) Erfinder: Schwienbacher, Helmut, 39100 Bozen (IT)
(74) Vertreter: Dey, Michael

(57) **Zusammenfassung**

Durch technologische und wirtschaftliche Zusammenlegung zweier an sich getrennter Anlagen - einer Photobioanlage und einer Biogasanlage - zu einem Anlagenverbund wird die kostengünstige Herstellung von Ölen aus Algen ermöglicht.

## Beschreibung

Die Erfindung betrifft Verfahren und Vorrichtungen zur Herstellung von Bioöl aus Algen.

Algen sind aufgrund Ihrer Fähigkeit, Photosynthese zu betreiben, potentielle Lieferanten großer Mengen pflanzlicher Biomasse. Elektromagnetische Energie in Form von Licht wird von Algen unter Verwendung von Farbstoffen absorbiert, in chemische Energie umgewandelt und diese Energie bei Gegenwart von CO₂ und Wasser zum Aufbau und zur Synthese von organischen Verbindungen und für das Wachstum verwendet. Bestimmte Algentypen besitzen außerdem in ihrer Biomasse große Anteile von Fetten bzw. Ölen.

Bei optimalen Kulturbedingungen vermehren sich die Algen etwa 50 bis 100 mal schneller als die wichtigsten heute bekannten Ölpflanzen. Zudem ist der Wasserverbrauch bei der Aufzucht von Algen vergleichsweise gering, und bei gleicher Anbaufläche lassen sich etwa 10 bis 100 mal höhere Erträge als mit den heute wichtigsten Ölpflanzen erzielen. Es wird deshalb in vielen Forschungsgruppen daran gearbeitet, durch Aufzucht von Algen kommerziell verwertbare Biomasse und insbesondere Bioöle herzustellen.

Mit den bis heute bekannten Verfahren und Anlagen ist es jedoch noch nicht möglich, Biomasse aus Algen in großem Maßstab so wirtschaftlich und preiswert herzustellen, dass das daraus gewonnene Öl oder Fett, als Energiequelle, zu den Pflanzenölen konkurrenzfähig verwendet werden kann.

Im Stand der Technik werden im Allgemeinen zwei verschiedene Verfahren und Produktionssysteme eingesetzt, um Algen zu züchten:

Die Zucht in offenen Becken wird vor allem in südlichen, sonnenreichen Gebieten, wie zum Beispiel in Kalifornien betrieben. Es ist aber nicht möglich die Algen auf eine bestimmte zum Beispiel hoch fetthaltige Spezies zu konzentrieren und zu beschränken. Fremdkontamination durch Pilze und Bakterien erfolgt, die Ausbeute erreicht auch wegen der geringen Eindringtiefe der Sonnenenergie kaum 10 bis 20 g Biomasse pro Tag und m² Biofläche. Fetthaltige Algensorten werden von schneller wachsenden Arten kurzfristig verdrängt. Hochfetthaltige Algen, die für die Energiegewinnung vorteilhaft sind, können nicht produziert werden. Diese Systeme sind außerdem in Europa wegen der klimatischen Bedingungen im Regelfall nicht einsetzbar und für den praktischen Dauerbetrieb zur Energieerzeugung nicht geeignet.

Geschlossene Systeme, so genannte Photobioreaktoren, gibt es in den verschiedensten Ausführungen. Meist als Rohrsysteme in südlichen Regionen im Freien und daher mit geringer Winterausbeute. In mittleren geographischen Breitengraden ebenfalls als Rohrsysteme in Glashäusern eingehaust und mit zu geringer Lichtausbeute und hohen Heizkosten, um die Anlagen in Winter betreiben zu können. Es sind auch Plattenreaktoren bekannt, diese sind meist ebenfalls nicht wetterfest, meist auch in Glashäusern platziert und nicht für den Winterbetrieb ausgelegt. Die heute bekannten Modelle erreichen Ausbeuten pro Tag von 40 - 50 g Trockenmasse/ m² Photofläche. Sie sind in der Anschaffung teuer, müssen beheizt werden, und CO₂ und die teuren Nährstoffe müssen zugekauft werden.

Einige dieser Reaktormodelle sind auch an Energiezentralen angebunden, um preiswert Wärme und CO₂ zur Verfügung zu stellen. Doch allen bis heute bekannt gewordenen Modellen fehlen jene entscheidenden technischen Vorrichtungen, die Voraussetzung sind, um eine konkurrenzfähige Wirtschaftlichkeit bei der Produktion von Biomasse mit hohem Fettgehalt verlässlich erreichen zu können.

Die Aufgabe der vorliegenden Erfindung war es daher, die vorstehend geschilderten Kernprobleme der bisher bekannten Algenzuchtanlagen, eine aufwändige Klimatisierung, eine nicht konstante Leuchtdichte, zu geringe Ausbeuten, hohe Kosten für das CO₂ und für die Nährstoffe, zu hohe Bau- und Betriebskosten, auszuschalten.

Die Lösung dieser Problematik wird erfindungsgemäß durch das Zusammenschmelzen von zwei üblicherweise getrennt geführten Anlagen, einer Biogasanlage und eines Photobioreaktors, durch neue Technologien und durch den Aufbau eines vielfältigen Verbundkreislaufes an Rohstoffen und Betriebsmitteln zwischen beiden Anlagen bereitgestellt, wodurch eine besonders hohe Wirtschaftlichkeit erreicht werden kann. Außerdem können durch die erfindungsgemäßen Vorrichtung und Verfahren die bisher bekannten Ausbeuten an Algenbiomasse bei sehr geringen Produktionskosten deutlich gesteigert werden.

Die Erfindung stellt in einer ersten Ausführungsform eine Vorrichtung zur Herstellung von Bioöl bereit, umfassend eine Biogasanlage und einen Photobioreaktor, worin der Photobioreaktor umfasst:
- einen oder mehrere Behälter mit einem Substratgemisch, umfassend Algen, Nährstoffe, Wasser und ggf. weitere Zusatzstoffe, die in
- einem oder mehreren Flüssigkeitskreisläufen mit Photokompartimenten verbunden sind, in denen die Algen Licht ausgesetzt werden können;
und worin die Biogasanlage und der Photobioreaktor derart miteinander verbunden sind, dass in der Biogasanlage erzeugte Produkte, insbesondere Gärsubstrat, CO₂, und/oder Abwärme, dem Photobioreaktor zugeführt werden können.

Durch die Integration eines Photobioreaktors mit einer Biogasanlage wird ein entscheidender Beitrag zur Rentabilität erreicht. Die dabei entstehenden Synergien und Kostensenkungen ermöglichen, dass mit der erfindungsgemäßen Vorrichtung die Herstellung von Bioöl aus Algen unabhängig vom Standort und von der Jahreszeit in zuvor nicht erreichter Menge und bei niedrigen Herstellungskosten möglich ist, sodass die gewonnenen Bioöl-Produkte konkurrenzfähig zu den heute am Markt befindlichen Pflanzenölen sind.

Der Aufbau eines Wärmeverbundes zwischen der Biogasanlage und dem Photobioreaktor ermöglicht, die Abwärme der Biogasanlage zur Erwärmung und Klimatisierung des Photobioreaktors zu nutzen. Die Dimensionierung der Biogasanlage und des Photobioreaktors ist dabei vorzugsweise so ausgelegt, dass die von der verbundenen Biogasanlage erzeugte Abwärme zur Klimatisierung und für den gesamten Wärmebedarf des Photobioreaktors ausreicht. Der erfindungsgemäße Photobioreaktor ist in einer bevorzugten Ausführungsform so konzipiert, dass gewährleistet ist, das gesamte System leicht und preiswert gegen Kälte zu isolieren, und zu jeder Jahreszeit auf konstanter und wählbarer Temperatur zu halten.

Das von der Biogasanlage produzierte CO₂ kann den Algen in dem Photobioreaktor für die gewünschte Photosynthese zur Verfügung gestellt werden. Vorzugsweise wird das CO₂ zuvor gereinigt und entölt, beispielsweise unter Verwendung einer Aktivkohlereinigung. Die erfindungsgemäße Vorrichtung zur Herstellung von Biogas umfasst daher vorzugsweise Mittel für die Reinigung von CO₂ aus dem Gas der angeschlossenen Biogasanlage und für die Zufuhr des gereinigten CO₂ in den Photobioreaktor. In einer bevorzugten Ausführungsform sind für die CO₂-Zufuhr Injektoren vorgesehen, die jeweils am Eingang der Photokompartimente angebracht sind. Über die Injektoren kann CO₂ in Pulsen in das vorbeiströmende Substratgemisch injiziert werden, wobei z.B. zwischen 1 bis 200 Takte pro Minute verwendet werden können. Die pulsweise Injektion bewirkt neben einer Erhöhung der Löslichkeit des CO₂ in Wasser durch den herrschenden hydrostatischen Druck auch, dass die Algen kontinuierlich von dem CO₂ umspült und durch die Vibrationsbewegungen zur Aufnahme von CO₂ stimuliert werden. Der pulsierende Eintrag hat Wirkungen ähnlich einem schonenden Rührsystem und erfolgt so, dass die Zellkörper nicht angegriffen oder beschädigt, sondern lediglich stimuliert werden.

Der Nährstoffbedarf der Algen kann ebenfalls zumindest teilweise durch den Gärsubstrataustrag der Biogasanlage gedeckt werden. Nährstoffe im Sinne der Erfindung umfassen Stickstoff, Phosphor, Kalium und Spurenelemente. Vor der Zufuhr des Gärsubstrats der Biogasanlage zu dem Photobioreaktor wird dieses vorzugsweise mittels chemischer und/oder physikalischer Konditionierung bei Temperaturen zwischen etwa 25 °C und 100 °C aufbereitet. In einer bevorzugten Ausführungsform umfasst die erfindungsgemäße Vorrichtung zur Herstellung von Biogas daher Mittel für die chemische und/oder physikalische Behandlung des Gärsubstrats der Biogasanlage und für die Zufuhr des behandelten Gärsubstrats in den Photobioreaktor. Durch ein dem jeweiligen flüssigen Gärsubstrat der Biogasanlage angepasstes Verfahren wird der Flüssigaustrag so aufbereitet, dass alle für das Algensubstrat des Bioreaktors notwendigen Nährstoffe gewonnen und wieder verwertet werden können. Durch diese Vorgehensweise wird es möglich, aus dem Austrag der Biogasanlage, den gesamten Bedarf der Algen an Stickstoff, Phosphor, Kalium und der meisten Spurenelemente vollständig abzudecken.

Die aus dem Austrag der Biogasanlage gewonnene Nährlösung wird je nach Bedarf mit weiteren Nährstoffen ergänzt und in einem oder mehreren Lagerbehältern sterilisiert und zwischengelagert. Es wird so eine weitgehende Sterilisation des einzubringenden Substrates erreicht und eine Kontamination der eingesetzten Nährlösungen durch fremde Algenstämme, durch Pilze und Bakterien vermieden. Dadurch kann eine störungsfreie Betriebsführung und gute Ausbeuten an hoch fetthaltigen Algentypen erreicht werden, da diese sich sonst leicht von schneller wachsenden Algenstämmen verdrängen lassen.

Eine wesentliche Nährstoffkomponente für das Wachstum von Algen ist Stickstoff. Dieser muss daher in den zur Verfügung gestellten Nähstoffen in relativ hoher Konzentration enthalten sein. Es wurde festgestellt, dass Algen bei Kultivierung unter Stickstoffmangel in eine Stresssituation versetzt werden. Sie beginnen dann, Vorräte in Form von Fetten anzulegen und zeigen eine erhöhte Fettproduktion. Um diesen Effekt zu nutzen umfasst die erfindungsgemäße Vorrichtung zur Herstellung von Bioöl in einer bevorzugten Ausführungsform Mittel für die Steuerung/Regelung der Nährstoffzufuhr, insbesondere der Stickstoffzufuhr in den Photobioreaktor. So ist es möglich, die Algen gezielt in eine Stresssituation zu versetzen, und zur erhöhten Fettproduktion anzuregen. Durch eine richtig gesteuerte systematische zyklische Stickstoffkarenz kann der prozentuale Anteil an Fetten in der Trockenmasse der Algen gesteigert werden ohne wesentliche Auswirkungen auf die Produktivität. Entsprechend den biologischen Bedürfnissen liegen die Zyklen bei etwa 1-15 Tagen, d.h. die durch Stickstoffmangel hervorgerufene Stresssituation wird kontinuierlich und zyklisch über 1 bis 15 Tage aufrecht erhalten und wieder aufgehoben.

Ein weiterer Vorteil der erfindungsgemäßen Integration eines Photobioreaktors und einer Biogasanlage besteht darin, dass der Betreiber Anspruch auf den im EEG (Elektroeinspeisungsgesetz) vorgesehenen Wärme- und Innovationsbonus und damit Kapital- und Wartungskosten für die Algenanlage abgedeckt werden können. Abgesehen von den Kapital- und Wartungskosten, können die aus den Algen erwirtschafteten Erlöse, die Material- und Betriebskosten bei weitem abdecken.

In einer besonders bevorzugten Ausführungsform umfasst die erfindungsgemäße Vorrichtung weiterhin Mittel für die Rückführung von Algenbiomasse, die in dem Photobioreaktor produziert und ggf. für die Bioölherstellung verwendet wurde, in die Biogasanlage. Die von dem Photobioreaktor erzeugten Biomassen können dadurch, direkt oder nach zuvoriger Abtrennung der Bioöle aus den Algen, wieder als Rohstoffe in der Biogasanlage eingesetzt werden. Durch diese Rückführung werden weitere Einsparungen an Material- und Betriebskosten erreicht.

In der erfindungsgemäßen Vorrichtung zur Herstellung von Bioöl sind ein oder mehrere Behälter mit dem Substratgemisch, welches Algen, Nährstoffe, Wasser und ggf. weitere Zusatzstoffe umfasst, in ein oder mehreren Flüssigkeitskreisläufen mit Photokompartimenten verbunden, in denen die Algen Licht ausgesetzt werden können. Die Photokompartimente sind vorzugsweise aus einem UV-beständigen Material ausgebildet. Beispiele für geeignete Materialien sind durchsichtige Materialien wie z.B. Glas oder Kunststoff wie Acrylate, Polycarbonat oder PET. Form und Größe der Photokompartimente sind grundsätzlich nicht eingeschränkt. Sie können beispielsweise lichtdurchlässige Röhren oder Platten umfassen. Die Dimensionen von Platten können bei etwa 1 bis 10 m Höhe und 1 bis 10 m Länge und einer Schichtdicke von 1 bis 35 cm liegen. Beispielsweise kann eine Platte eine Höhe von etwa 3 m und eine Länge von etwa 3 m aufweisen, sowie eine Schichtdicke von 1-15 cm, bevorzugt 3-10 cm. Bevorzugt sind mehrere Platten in Reihe geschaltet, und in einem oder mehreren geschlossenen Flüssigkeitskreisläufen mit dem Behälter verbunden sind.

Beim Umlauf nimmt die Aufwuchsdichte der Algen im Substratgemisch rasch zu. Es wurde erfindungsgemäß festgestellt, dass zur Beibehaltung optimaler Wachstumsbedingungen in den gesamten Umlaufzyklen der Algen, im stromabwärtsgelegenen Teil des Kreislaufs daher eine verstärkte turbulente Strömung vorteilhaft ist. Dies kann in dem erfindungsgemäßen Photobioreaktor dadurch erreicht werden dass die Schichtdicke der stromabwärts gelegenen Platten verringert ist. Während im ersten Teil des Kreislaufs Platten mit Schichtdicken von z.B. 3 - 12 cm verwendet werden, kommen im zweiten Teil bevorzugt Schichtdicken von 1 - 8 cm, vorzugsweise zwischen 3 - 6 cm zum Einsatz. Die Durchflussmenge wird nicht verändert, so dass durch Verringerung des Plattenquerschnitts die Fließgeschwindigkeit und damit auch die Turbulenz ansteigen.

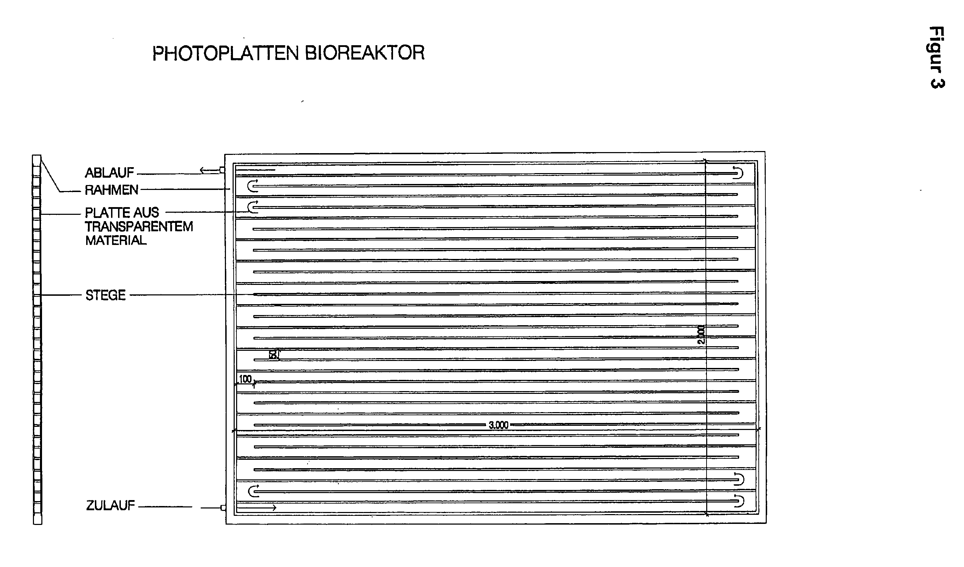

Beim Einsatz von Platten als Photokompartimente sind diese bevorzugt innen mit Trennleisten, auch Stege genannt, als Flüssigkeitsführungen versehen, die einen Labyrinthkanal bilden durch welchen das Substratgemisch gepumpt und den Lichtstrahlen ausgesetzt werden kann (siehe Figur 3). Die Stege als Flüssigkeitsführungen können beispielsweise aus Glas oder Kunststoff ausgebildet sein. Vorzugsweise sind sie parallel zueinander im Abstand von z.B. 2 - 20 cm angebracht.

Der erfindungsgemäße Photobioreaktor ist vorzugsweise so konzipiert, dass die Photokompartimente über den gesamten Tag und das ganze Jahr mit einer gleichbleibenden und biologisch optimalen Lichtintensität belichtet sind.

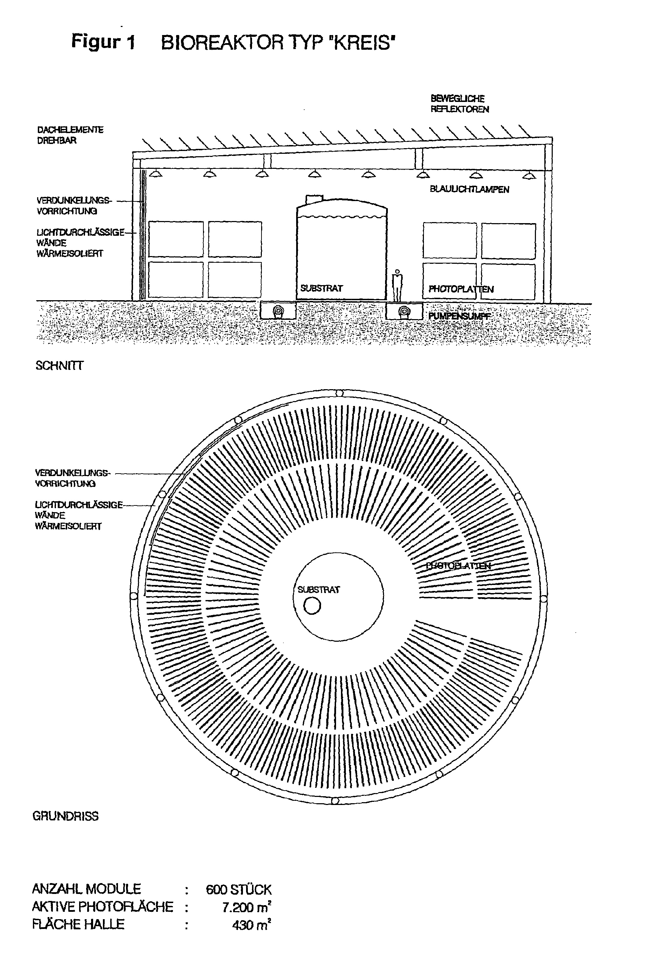

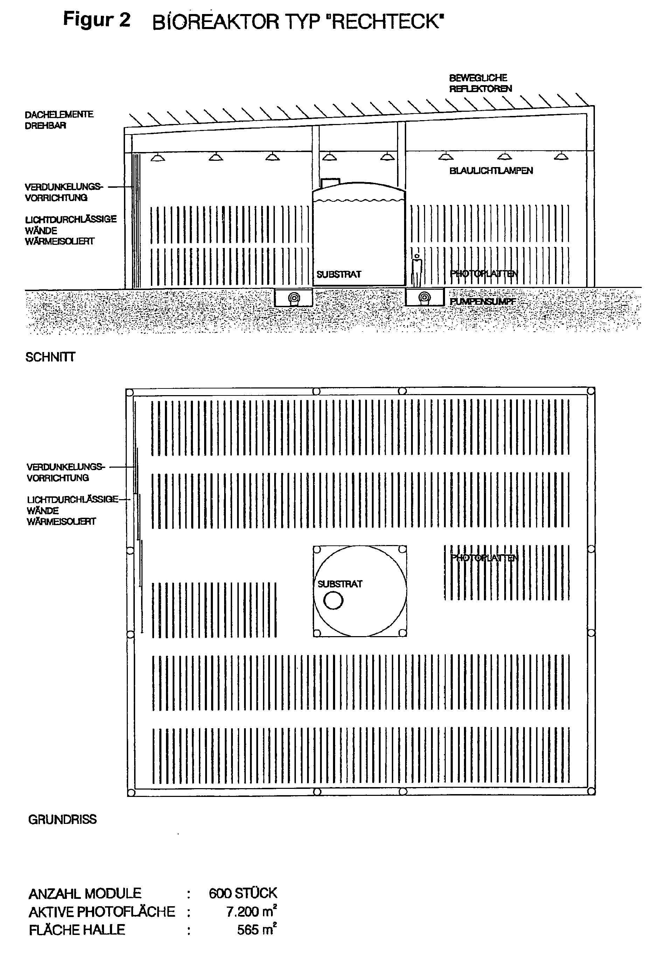

Eine bevorzugte Ausführungsform einer erfindungsgemäßen Vorrichtung ist in den Figuren 1 und 2 veranschaulicht.

Das Dach des Photobioreaktors ist aus geeigneterweise aus lichtdurchlässigen, wärmeisolierenden Materialien ausgeführt. Ebenfalls bestehen alle Außenwände vorzugsweise aus leicht abnehmbaren, voll lichtdurchlässigen und wärmeisolierten Materialien. Die gesamte Anlage kann mit der Abwärme der Biogasanlage ganzjährig durch Kühlung oder Heizung auf einer Temperatur, die zwischen 18° C und 35° C liegen kann, gehalten werden. Die Verluste an Wärme können durch die kompakte Bauweise minimiert werden.

Über der Dachisolierung ist z.B. eine Shetdach-ähnliche Konstruktion mit Jalousien aufgebaut. Diese Jalousien sind um einen Winkel von bis zu 180° drehbar, sie können dem Lauf der Sonne folgen und sie sind auch in der Neigung so verstellbar, dass ganzjährig durch Ausrichtung des Solardaches zur Sonne und durch Einstellung der Neigungsflächen das Sonnenlicht senkrecht in den Photoraum fällt. Die reflektierenden Oberflächen des Shetdaches und der Jalousien garantieren einen optimalen Lichteintrag und damit maximales Wachstum über 12 Monate /Jahr.

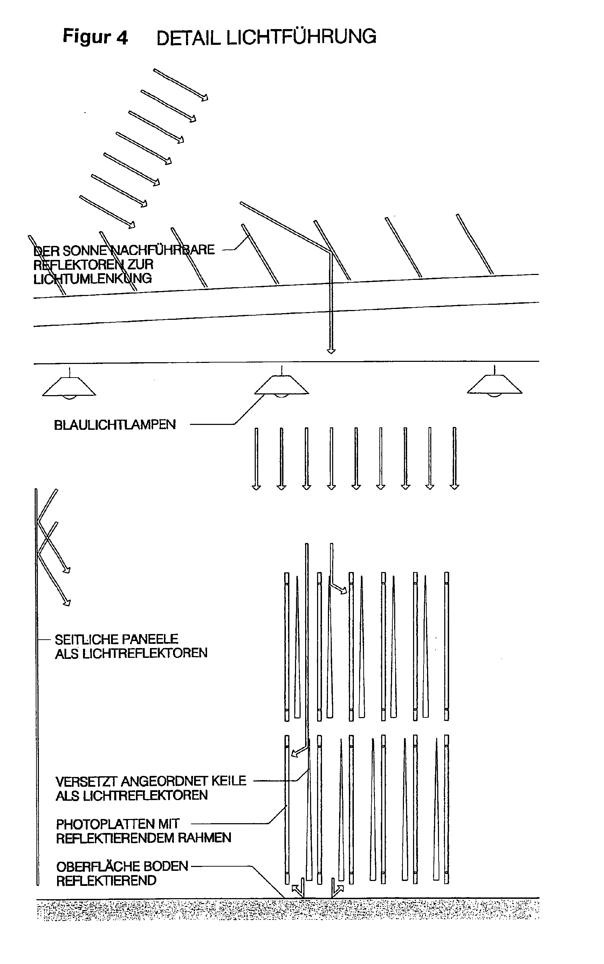

Um eine optimale Leuchtdichte auf alle Photokompartimente zu erreichen, sind in den Zwischenräumen in einer bevorzugten Ausführungsform reflektierende Elemente wie z.B. Keile, in genügender Anzahl und mit geeigneter Bauform so angebracht, dass durch Spiegelung die senkrecht einfallenden Lichtstrahlen umgelenkt und auf die Photokompartimente geleitet werden und so eine gleichmäßige Beleuchtung der gesamten Flächen der Photokompartimente ermöglicht wird. Durch Einbau von zusätzlichen reflektierenden Leisten und Flächen am Boden und an den Außenseiten und durch die reflektierende Ausführung der Rahmen der Photokompartimente können die direkten Sonnenstrahlen durchgehend mit größter Ausbeute auf die gesamten Photooberflächen verteilt einwirken. (siehe Figur 4)

Der Photobioreaktor ist an den Außenseiten vorzugsweise mit lichtdurchlässigen, UV-stabilen, wärmeisolierenden und abnehmbaren Abdeckungen versehen. Diese können z.B. aus Glas, Acrylglas, Polycarbonat oder anderen transparenten Materialien bestehen.

Das gesamte Photoareal erhält so sowohl über die Außenwände als auch von oben die benötigte Lichtenergie.

Der Energieeintrag in die Photokompartimente erfolgt über das natürliche Tages- und Sonnenlicht. Um täglich über 12 h und über das gesamte Jahr die von den Algen aufnehmbare maximale "Photosynthetisch nutzbaren Strahlenanteile (PAR -Photosynthetic Active Radiation)" zu gewährleisten und den Algen zur Verfügung zu stellen, ist Tageslicht allein in verschiedenen Monaten als Lichtquelle nicht ausreichend. In den Wintermonaten und an trüben Tagen wird nicht immer über die gesamte Tageszeit die für das optimale Wachstum notwendige Strahlung erreicht. Um die für die Photosynthese nutzbaren Strahlenanteile (PAR) immer optimal zu erzeugen, werden daher vorzugsweise Lichtquellen mit Kunstlicht in die Anlage integriert, um die fehlenden Strahlungsanteile auszugleichen. Teil dieser Erfindung ist auch der dazu eingesetzte Lampentyp. Verwendet werden bevorzugt Kunstlichtlampen, die natürliches Licht zwischen 400 und 700 nm ausstrahlen und außerdem einen erhöhten Anteil an Blaulicht (ca. 420-490 nm) abgeben. Es wurde überraschenderweise festgestellt, dass durch Verwendung von Lampen mit erhöhtem Blauanteil das Wachstum der Algen gesteigert werden kann, zudem entwickelt sich die Algenmasse bei hohem Blaulichtanteil mit einer viel kompakteren Konsistenz und Dichte.

Bei der Photosynthese wird Lichtenergie von der Pflanze und auch von der Alge aufgenommen und zur chemischen Synthese verwendet.

Die brutto Reaktionsgleichung bei der Photosynthese ist

6 CO₂ + 12 H₂O = C₆H₁₂O₆ + 6 O₂ + 6 H₂O.

Aus CO₂ und Wasser bildet sich in der ersten Zwischenstufe ein Zucker. Dabei wird eine Veränderung des Energieinhaltes in dem System erreicht. Die Zunahme des Energieinhaltes beträgt 2870 kJ x Mol⁻¹.

Diese chemische Umsetzung läuft nur dann optimal ab, wenn eine bestimmte und genügend hohe Lichtdichte zur Verfügung steht. Diese Lichtdichte, die so genannten für die Photosynthese nutzbaren Strahlenanteile (PAR), müssen kontinuierlich in der richtigen Dosierung zur Verfügung stehen. Eine zu hohe Dichte führt zu Chlorophyll-Wucherungen und einer Verminderung der Eindringtiefe des Lichtes. Eine zu geringe Dichte reduziert die Photosynthese und somit das Wachstum entscheidend. Mit einer Lichtstärke von mindestens 80 bis 100 % des PAR werden die besten Wachstumsergebnisse erreicht. Diese optimale Bestrahlung wird durch das erfindungsgemäße Belichtungssystem zusammen mit der hier nachstehend beschriebenen biologischen Steuerung kontinuierlich erreicht und eingehalten. Je nach Notwendigkeit und den momentanen Lichtverhältnissen angepasst können Natur- und Kunstlicht kombiniert eingesetzt werden.

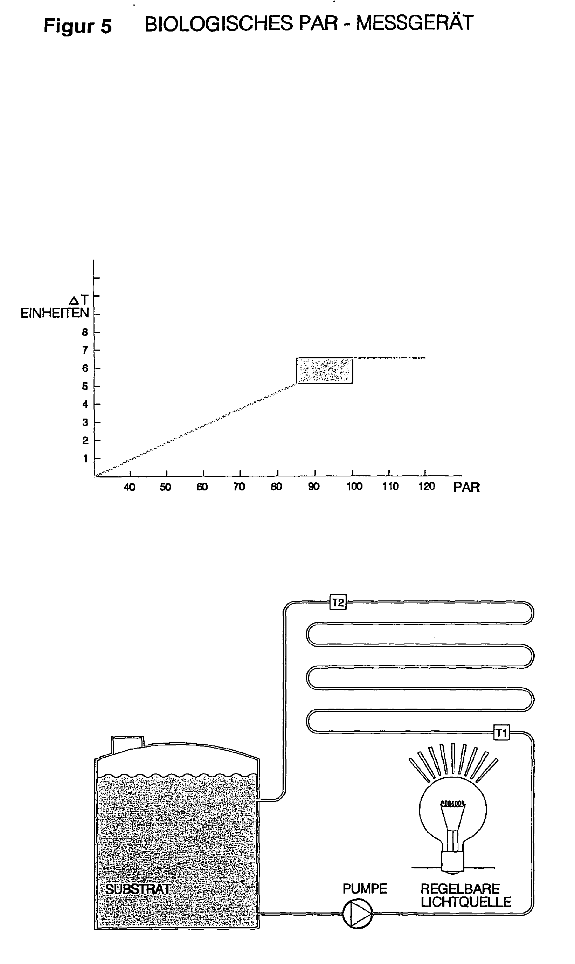

Die Steuerung der Lichtdichte erfolgt vorzugsweise über eine automatische biologische Steuerung. Dazu kann ein biologisches Mess- und Steuergerät in mehreren Einheiten in der Vorrichtung aufgebaut und zur automatischen Dosierung und einer dezentralen Steuerung der Lichtenergie verwendet werden (siehe Figur 5). Das Messgerät nutzt den messbaren sehr kleinen Temperaturanstieg, der erfolgt wenn ein mit Algensubstrat durchflossener Bauteil, z.B. ein transparentes Rohr, von Lichtenergie bestrahlt wird. Bei einer Steigerung der Strahlungsenergie steigt auch die Temperatur an. Diese erreicht ein Maximum, wenn ca. 100 % des PAR eingetragen werden und bleibt bei diesem Wert auch wenn die Strahlungsintensität noch wesentlich weiter gesteigert wird. Mit dem höchsten Temperaturanstieg ist die maximale photosynthetisch nutzbare Lichtenergie erreicht und angezeigt. Ein höherer Eintrag kostet Energie und führt zu Wachstumsstörungen.

Durch diese biologischen Messsysteme können dezentral die optimalen Licht- und Betriebsbedingungen ermittelt und eingehalten werden und die besten Kombinationen von Natur- und Kunstlicht automatisch eingestellt werden. Durch diese Lichtführung kann eine optimale Lichtdichte nahezu überall und dauernd erreicht und gehalten werden. Verglichen mit der üblicherweise verwendeten diffusen Einstrahlung kann so die Ausbeute an Algenbiomasse auch bei alleinigem phototrophen Wachstum deutlich gesteigert werden. Durch die kontinuierliche hohe Lichtzufuhr ist das für Öl produzierende Algen notwendige hohe Energieniveau zur enzymatischen Fettsynthese an allen Aufwuchsflächen kontinuierlich verfügbar und es werden so die besten Randbedingungen für eine Steigerung des Fettgehaltes in den Algen eingehalten und gewährleistet.

In einer bevorzugten Ausführungsform ist der erfindungsgemäße Photobioreaktor so konzipiert, dass die Photokompartimente abwechselnd belichtet und verdunkelt sind. Durch zu hohe Lichtdichte erhalten die Zellen an der Oberfläche mehr Photonen als zur physiologischen Photosynthese benötigt werden. Die Strahlen werden von Chlorophyll trotzdem absorbiert und die Farbpigmente beginnen zu wuchern und können das Eindringen der Strahlen in die tieferen Schichten reduzieren.

Es wurde festgestellt, dass die alternierende Exposition des Algensubstrates an Licht und Dunkelheit zu wesentlich stabilerem und stärkerem Wachstum der Algen führt. Dies ist auch physiologisch nachvollziehbar, denn in der Dunkelperiode wird den Zellen die Zeit gegeben, die von den Farbpigmenten absorbierte Lichtenergie für die nachfolgenden biologischen und chemischen Aufbausynthesen weiter zu verwerten. Ein Licht Dunkel Verhältnis mit Zeitintervallen zwischen 3-20 min führt zu einem Optimum des Wachstums bei bester Stabilität und geringster Wachstumsoszillation der Algen. Auch kann durch diese Maßnahme eine Steigerung der Fettgehalte erreicht werden. Vorzugsweise werden als die besten Licht - Dunkel Effekte Zyklen von 10 - 14 min ermittelt.

Die vorliegende Erfindung stellt daher zwei verschiedene unabhängige Vorrichtungen für die technische Durchführung der Licht-Dunkel Zyklen bereit, die vorzugsweise zur besseren Steuerung der Anlage in den Photobioreaktor integriert sind.

Das (Shet)-Dach und die gesamte Außenhülle des Photobioreaktors sind so gestaltet, dass sie innerhalb kurzer Zeit, etwa innerhalb einer Minute, lichtdicht geschlossen oder geöffnet werden. Es kann so volle Dunkelheit im gesamten Reaktorraum erreicht und für einen vorgegebenen Zeitraum eingehalten werden.

Außerdem können die verschiedenen Kreislaufsysteme, in welchen das Substrat vom dunklen Vorratsbehälter über Pumpen in die Photokompartimente fließt und von dort wieder zurück in den Vorratsbehälter so gewählt werden, dass die Verweilzeiten im Licht bei der Kreislaufführung der einzelnen Pumpensegmente variabel einstellbar sind und so ebenfalls automatisch ein Hell-Dunkel Effekt zwischen 1 bis 40 Minuten eingestellt werden kann. Beispielsweise kann die Umlaufzeit des Algensubstrates vom Lagerbehälter, über die Photokompartimente bis zum Rückfluss in den Behälter so gewählt werden kann, dass ein vollständiger Kreislauf variabel zwischen 1 und 40 Minuten möglich ist.

Der/die Behälter in dem das Substratgemisch vorliegt kann zentral in der Mitte des Photobioreaktors oder auch dezentral platziert werden. Seine Kapazität entspricht dem je nach Baugröße benötigten Substratvolumen; dieses liegt z.B. bei circa 5 bis 15 l, vorzugsweise aber bei ca. 10 l pro m² Photofläche. Als Photofläche wird die mit Licht bestrahlbare Oberfläche der Photokompartimente bezeichnet.

Das Füllvolumen und die Bauhöhe des Behälters ist vorzugsweise so ausgelegt, dass der Flüssigkeitsspiegel im Behälter immer über dem höchsten Punkt der Photokompartimente liegt und so keine hydrostatische Höhendifferenz beim Umpumpen zu überwinden ist, die Anlage dadurch höchst energiesparend betrieben und den Algenzellen keine Beschädigungen zugefügt werden können.

Der Substratbehälter ist geeigneterweise mit einer Servicestation zum Ernten der Algen und zur Regenerierung der Substratlösung verbunden.

Um den Substratbehälter sind die Photoflächen z.B. strahlenförmig nach außen, im Kreis oder im Rechteck oder in einer anderen Form angeordnet. Mit Pumpen am Fuße des Behälters werden die algenhaltigen Substrate durch die Photokompartimente und wieder in den Behälter zurückgeführt.

Die Fliessgeschwindigkeit in den Photokompartimenten sollte immer ausreichend hoch sein, um Ablagerungen an den Photoflächen zu vermeiden. Bevorzugt liegt sie über 0,5 m pro Sekunde. Außerdem werden in einer bevorzugten Ausführungsform Reinigungselemente wie Kugeln oder andere Festkörper aus z.B. PE oder PVC oder aus anderen Materialien wie beispielsweise Glas mit einem Durchmesser oder Kantenlängen von z.B. 2 - 7 mm in genügender Menge zugegeben, um die Photoflächen kontinuierlich zu reinigen und besatzfrei zu halten.

Zur besseren Verwirbelung der Biomasse und zur stärkeren Turbulenzbildung weisen die von dem Substartgemisch durchströmten Bereiche der Photokompartimente in einer bevorzugten Ausführungsform Rillen auf. Beispielsweise können in den Flüssigkeitsführungen der Platten oder in den Wänden der Rohre Rillen eingearbeitet sein. Durch die erhöhte Turbulenz wird einerseits eine zu rasche Lichtsättigung des Algensubstrates vermieden und zum anderen wird die Reinigungswirkung der vorstehend beschriebenen Reinigungselemente verstärkt.

Bei Einsatz von CO₂ und Licht können mit der erfindungsgemäßen Vorrichtung im Schnitt bereits sehr hohe Ausbeuten an Biomasse in g/Tag / m² Photofläche erreicht werden. Weitere Verbesserungen dieser Erträge können in einer bevorzugten Ausführungsform des erfindungsgemäßen Verfahrens durch eine gemischt photo- und autotrophe Fahrweise erreicht werden. Dabei werden Zusätze in das Substratgemisch von organischen Substanzen in einer Konzentration von vorzugsweise 200 bis 4000 mg/l CSB hinzugefügt. Von den verschiedenen erprobten organischen Substanzen haben sich die ein- und mehrwertigen Alkohole, vor allem Methanol, Glykole oder Glycerin, sowie Fettsäuren und Ester am besten bewährt.

In einer weiteren Ausführungsform stellt die vorliegende Erfindung ein Verfahren zur Herstellung von Bioöl bereit, umfassend die Schritte:
(a) Bereitstellen eines Substratgemischs, umfassend Algen, Nährstoffe, Wasser und ggf. weitere Zusatzstoffe,
(b) Zirkulieren des Substratgemischs durch Photokompartimente, in denen die Algen Licht und CO₂ ausgesetzt werden, um Photosynthese zu ermöglichen,
(c) Isolieren von Algen aus dem Substratgemisch,
(d) Gewinnen von Bioöl aus den isolierten Algen,
worin eine vorstehend beschriebene Vorrichtung verwendet wird.

Die Verfahrensführung erfolgt vorzugsweise wie vorstehend im Zusammenhang mit der erfindungsgemäßen Vorrichtung beschrieben.

Das Substratgemisch wird in Schritt (a) in einem oder mehreren Behältern zur Verfügung gestellt. Die Behälter sind vorzugsweise lichtundurchlässig, so dass in diesen Behältern keine Photosynthesereaktion ablaufen kann. Das Substratgemisch wird aus den Lagerbehältern z.B. über Pumpensysteme in geeignete Photokompartimente geleitet, in denen das Substratgemisch Licht und CO₂ ausgesetzt ist, und zirkuliert schließlich zurück in die Ausgangsbehälter. Während der Einwirkung von Licht wird durch Photosynthese Biomasse (Algen) erzeugt. Diese Algen werden in Schritt (c) über geeignete Systeme aus dem Substratgemisch isoliert, und in Schritt (d) wird aus den Algen Bioöl gewonnen.

In einer bevorzugten Ausführungsform des erfindungsgemäßen Verfahrens werden die in Schritt (c) gewonnenen Algen und oder die Rückstände der Algen nach der Bioölgewinnung in Schritt (d), als Substrat in eine Biogasanlage zugeführt.

Das in Schritt (a) verwendete Substratgemisch kann weiter organische Substanzen wie ein- oder mehrwertige Alkohole, insbesondere Methanol, Glykole, Glycerin, Fettsäuren und/oder -ester, vorzugsweise in einer Konzentration von 200 - 4000 mg pro Liter CSB enthalten.

Die Fließgeschwindigkeit des Substratgemischs wird vorzugsweise in Abhängigkeit von der Bewuchsdichte der Algen variiert und beträgt bevorzugt mindestens 0,5 m/s beträgt.

Die Lichtmenge, die den Algen zur Verfügung gestellt wird, wird vorzugsweise so reguliert, dass sie etwa 80-100% der maximal aufnehmbaren photosynthetisch aktiven Strahlung (PAR) entspricht. Die von dem Photobioreaktor maximal aufnehmbare Lichtenergie zur Erzeugung von Biomasse kann wie vorstehend beschrieben über ein oder mehrere Temperaturmesseinrichtungen ermittelt werden, und der ermittelte Wert wird dann zur Steuerung des Lichtes der Anlage verwendet.

### Figurenlegende

Figur 1: Bioreaktor Typ "Kreis"
Figur 2: Bioreaktor Typ "Rechteck"
Figur 3: Photoplatten Bioreaktor
Figur 4: Detail Lichtführung
Figur 5: Biologisches PAR - Messgerät

## Patentansprüche

1. Vorrichtung zur Herstellung von Bioöl, umfassend eine Biogasanlage und einen Photobioreaktor,
worin der Photobioreaktor umfasst:
einen oder mehrere Behälter mit einem Substratgemisch, umfassend Algen, Nährstoffe, Wasser und ggf. weitere Zusatzstoffe,
die in einem oder mehreren Flüssigkeitskreisläufen mit Photokompartimenten verbunden sind, in denen die Algen Licht ausgesetzt werden können;
und worin die Biogasanlage und der Photobioreaktor derart miteinander verbunden sind, dass in der Biogasanlage erzeugte Produkte, insbesondere Gärsubstrat, CO₂, und/oder Abwärme, dem Photobioreaktor zugeführt werden können.

2. Vorrichtung nach Anspruch 1,
weiterhin umfassend
(i) Mittel für die chemische und/oder physikalische Behandlung des Gärsubstrats der Biogasanlage und Mittel für die Zufuhr des behandelten Gärsubstrats in den Photobioreaktor und/oder
(ii) Mittel für die Reinigung von CO₂ aus dem Gas der Biogasanlage und Mittel für die Zufuhr des gereinigten CO₂ in den Photobioreaktor, vorzugsweise Düsen an den Photokompartimenten, durch welche CO₂ in das vorbeiströmende Substratgemisch injiziert werden kann.

3. Vorrichtung nach Anspruch 1 oder 2,
worin der Photobioreaktor eine Vorrichtung für die Steuerung/Regelung der Nährstoffzufuhr, insbesondere für die Stickstoffzufuhr umfasst.

4. Vorrichtung nach einem der Ansprüche 1 bis 3,
worin die Photokompartimente lichtdurchlässige Röhren und/oder ein oder mehrere Platten aus vorzugsweise UV-beständigem Material umfassen, worin die Platten in ihrem Inneren Trennleisten als Flüssigkeitsführungen aufweisen, die ein Kanalsystem ausbilden, durch welches das Substratgemisch geführt und Lichtstrahlen ausgesetzt werden kann.

5. Vorrichtung nach einem der vorhergehenden Ansprüche,
worin in Fließrichtung je am Anfang und am Ende der Photokompartimente Temperaturmesselemente vorhanden sind.

6. Vorrichtung nach einem der vorhergehenden Ansprüche,
worin das Substratgemisch weiterhin Reinigungselemente enthält, die dazu konzipiert sind, mit dem Gemisch durch die Photokompartimente zu zirkulieren und diese zu reinigen und besatzfrei zu halten.

7. Vorrichtung nach einem der vorhergehenden Ansprüche,
worin der Photobioreaktor weiterhin ein oder mehrere künstliche Lichtquellen, vorzugsweise mit erhöhtem Blaulichtanteil, umfasst, über welche die Photokompartimente belichtet werden können.

8. Vorrichtung nach einem der vorhergehenden Ansprüche,
worin der Photobioreaktor im Bereich der Photokompartimente Reflektionsflächen aufweist, die dazu konzipiert sind, eine gleichmäßige Beleuchtung der Photokompartimente zu gewährleisten.

9. Vorrichtung nach einem der vorhergehenden Ansprüche,
worin der Photobioreaktor Verdunkelungsvorrichtungen umfasst, die dazu konzipiert sind, die Photokompartimente wahlweise zu verdunkeln.

10. Verfahren zur Herstellung von Bioöl,
umfassend die Schritte:
(a) Bereitstellen eines Substratgemischs, umfassend Algen, Nährstoffe, Wasser und ggf. weitere Zusatzstoffe,
(b) Zirkulieren des Substratgemischs durch Photokompartimente, in denen die Algen Licht und CO₂ ausgesetzt werden, um Photosynthese zu ermöglichen,
(c) Isolieren von Algen aus dem Substratgemisch,
(d) Gewinnen von Bioöl aus den isolierten Algen
wobei das Verfahren in einer Vorrichtung nach einem der Ansprüche 1 bis 9 durchgeführt wird.

11. Verfahren nach Anspruch 10,
worin die Nährstoffe Stickstoff umfassen, und der Stickstoffgehalt in dem Substratgemisch in zyklischen Abständen variiert wird.

12. Verfahren nach Anspruch 10 oder 11,
worin die Nährstoffe Gärsubstrat aus einer Biogasanlage umfassen, welches vorzugsweise durch chemische und/oder physikalische Behandlung bei Temperaturen von insbesondere 25 bis 100°C aufbereitet, konditioniert und filtriert und ggf. sterilisiert wird, und dem Substratgemisch in Schritt (a) zugegeben wird, und/oder worin in Schritt (b) CO₂ aus dem Abgas der Biogasanlage, vorzugsweise gereinigtes CO₂, zugegeben wird..

13. Verfahren nach einem der Ansprüche 10 bis 12,
worin die Algen nach Schritt (c) oder die Rückstände der Algen nach Schritt (d), als Substrat in die Biogasanlage zugeführt werden.

14. Verfahren nach einem der Ansprüche 10 bis 13,
worin das Substratgemisch weiter organische Substanzen wie ein- oder mehrwertige Alkohole, insbesondere Methanol, Glykole, Glycerin, Fettsäuren und/oder -ester, vorzugsweise in einer Konzentration von 200 - 4000 mg pro Liter CSB enthält.

15. Verfahren nach einem der Ansprüche 10 bis 14,
worin die den Algen zur Verfügung stehende Lichtenergie in Schritt (b) so reguliert wird, dass sie etwa 80-100% der maximal aufnehmbaren photosynthetisch aktiven Strahlung (PAR) entspricht.
